# EUROPEAN PATENT SPECIFICATION

(11) **EP 1 642 589 B1**
(45) Date of publication and mention of the grant of the patent: **29.08.2012**
(21) Application number: 04733953.6
(22) Date of filing: 19.05.2004
(51) Int. Cl.: A61K 31/198, A61P 11/00, A61P 43/00, A23L 1/30, A23K 1/16

(54) **PREVENTIVES/REMEDIES FOR SNORE OR RESPIRATORY DISTURBANCES DURING SLEEP**
MITTEL ZUR PRÄVENTION/BEHANDLUNG VON SCHNARCHEN ODER ATEMSTÖRUNGEN IM SCHLAF
AGENTS PREVENTIFS/REMEDES POUR DES TROUBLES DU RONFLEMENT OU RESPIRATOIRES PENDANT LE SOMMEIL

(30) Priority: 19.05.2003 JP 2003141124
(43) Date of publication of application: 05.04.2006
(73) Proprietor: AJINOMOTO CO., INC., Chuo-ku Tokyo 104-8315 (JP)
(72) Inventor: SHIBAHARA, Susumu,c/o AJINOMOTO CO., INC.,, Kawasaki-shi, Kanagawa 210-8681 (JP); SAKAI, Ryosej,c/o AJINOMOTO CO., INC.,, Kawasaki-shi, Kanagawa 210-8681 (JP)
(74) Representative: Nicholls, Kathryn Margaret
(86) International application number: PCT/JP2004/007106
(87) International publication number: WO 2004/100989

(56) References cited:
- EP-A- 1 609 375
- WO-A2-00/51590
- WO-A2-03/015605
- CN-A- 1 159 919
- JP-A- 5 170 647
- JP-A- 57 193 417
- JP-A- 2002 504 510
- EGGERS E.D. ET AL: 'Mechanisms for the modulation of native glycine receptor channels by ethanol' J. NEUROPHYSIOL vol. 91, no. 6, June 2004, pages 2685 - 2695, XP002981827
- INOUE: 'Changes in tongue reflex responses during sleep' vol. 33, no. 1, 11 July 2003, pages 57 - 58, XP002981832
- DUTSCHMANN M. ET AL: 'Trigeminal reflex regulation of the glottis depends on central glycinergic inhibition in the rat' AM J PHYSIOL REGULATORY INTERGRATIVE COMP PHYSIOL vol. 282, no. 4, 2002, pages R999 - R1005, XP002981833
- KUBIN L. ET AL: 'Suppression of Hypoglossal motoneurons during the carbachol-induced antonia of REM sleep is not caused by fast synaptic inhibition' BRAIN RES. vol. 611, no. 2, 1993, pages 300 - 312, XP000609478
- HORNER R.L.: 'Is there a rationale in modulating brainstem neurons in obstructive sleep apnea and is it clinically relevant?' SLEEP vol. 23, no. 4, 2000, pages S179 - S181, XP002981834
- PATON J.F. ET AL: 'Central control of upper airway resistance regulating respiratory airflow in mammals' J. ANAT. vol. 201, no. 4, 2002, pages 319 - 323, XP002981835
- MORRISON J.L. ET AL: 'Glycine at hypoglossal motor nucleus: genioglossus activity, CO2 responses, and the additive effects of GABA' J. APPL. PHYSIOL. vol. 93, no. 5, 2002, pages 1786 - 1796, XP002981836
- SAARESRANTA T.: 'Hormones and Breathing' CHEST JOURNAL vol. 122, no. 6, 2002, pages 2165 - 2182, XP002981837
- YAMUY J. ET AL: 'Hypoglossal Motoneurons are postsynaptically inhibited during carbachol-induced rapid eye movement sleep' NEUROSCIENCE vol. 94, no. 1, 1999, pages 11 - 15, XP000922754
- KODAMA T. ET AL: 'Changes in inhibitory amino acid release linked to pontine-induced atonia: An in vivo microdialysis study' J. NEUROSCI. vol. 23, no. 4, February 2003, pages 1548 - 1554, XP002981838
- LAI Y-Y. ETAL: 'Changes in monoamine release in the ventral horn and hypoglossal nucleus linked to pontine inhibiton of muscle tone: An in vivo microdialysis study' THE JOURNAL OF NEUROSCIENCE vol. 21, no. 8, 2001, pages 7384 - 7391, XP002981839

## Description

### Technical Field

The present invention relates to an agent comprising glycine for the prophylaxis or treatment of snoring or a respiratory disorder during sleep, as well as a food and a diet effective for the prophylaxis or palliation of snoring or a respiratory disorder during sleep.

### Background Art

Snoring occurs when the airway is constricted or obstructed due to the decreased tension of the muscle of throat or tongue during sleep and the airway mucous membrane of the throat and the like vibrates. What causes snoring includes obesity, drinking, intake of hypnotic, aging, nasal diseases such as adenoid and the like, disorder in the throat and the like. Snoring not only disturbs housemate's sleep, thus causing trouble to others, but also prevents deep sleep and causes daytime sleepiness, decline of concentration, activity and memory, and mental instability. Therefore, prevention of snoring is important for ensuring good sleep of a housemate as well as the snorer, enhancing vitality during daytime and maintaining good health.

Approximately 10-20% of snorer shows an episode of repeated, intermittent sleep apnea (apnea during sleep) and such episode is clinically referred to as sleep apnea syndrome. Two million latent patients are estimated to be present in Japan.

As the etiology of sleep apnea, disorders in the respiratory central nervous system can be mentioned, besides the aforementioned airway obstruction.

It is known that snoring and sleep apnea not only prevent daytime activities but also cause traffic accidents due to daytime somnolence, and cause various disorders resulting from the effects of oxygen shortage in the body, which is caused by temporarily interrupted breathing, on the circulatory and central nervous systems. For example, association with hypertension, cerebral infarction, ischemic heart disease, arrhythmia and sudden death has been pointed out, and they are considered to be diseases that require prevention and treatment.

When snoring and sleep apnea are considered to be associated with obesity, drinking, continued use of hypnotic analgesic tranquilizer and the like, reform of lifestyle such as weight loss, nondrinking, withdrawal from hypnotic analgesic tranquilizer and the like becomes the primary treatment and prevention. For more positive treatment, use of intraoral accessory and surgical treatment by operation may be employed, though apnea may not be improved in some cases. For a more certain cure, a therapy by feeding air through a nose mask to always maintain positive pressure in the upper airway or CPAP (Continuous Positive Airway Pressure) has been recently developed, though compliance is not necessarily good.

As a treatment method using a pharmaceutical agent, drugs such as steroid etc. may be effective as far as snoring and sleep apnea are caused by inflammation of the airway or allergy. In addition, a hypotensive diuretic (acetazolamide) may be used for the treatment, but the treatment is not necessarily effective.

Moreover, there is a report indicating that pharmaceutical agents that suppress activities of glutamic acid neuron in the central nervous system, namely, inhibitors of glutamic acid receptor or glycine receptor, and antagonists of glutamic acid or glycine are potentially effective for the treatment of sleep apnea syndrome (WO00/51590), and the effectiveness of riluzole, which is a glutamic acid antagonist, was confirmed through animal experiments using rats. However, antagonists of glutamic acid or glycine having such efficacy have not been put to practical use in clinical situations and the effectiveness thereof in human has not been confirmed.

Therefore, effective therapeutic drugs, food and the like for snoring or sleep apnea have been demanded.

### Disclosure of the Invention

The problem to be solved by the present invention is provision of a pharmaceutical agent for the prophylaxis or treatment of respiratory disorders during sleep by preventing or reducing snoring, and food or diet for the prophylaxis or palliation of snoring or a respiratory disorder during sleep.

The present inventors have conducted intensive studies in an attempt to solve the above-mentioned problems and found that the administration of glycine suppresses occurrence of snoring, and further suppresses sleep apnea and reduced arterial blood oxygen saturation (SpO₂) due to sleep apnea.

Glycine can be added to a food or diet for utilization for the prophylaxis or palliation of snoring or a respiratory disorder during sleep.

Accordingly, the present invention provides the following.
1. An agent for use in the prophylaxis or treatment of snoring or a respiratory disorder during sleep, the agent comprising glycine or a salt thereof.
2. The agent for use as set out at 1, wherein the respiratory disorder during sleep is hypopnea or apnea.
3. The agent for use as set out at 2, wherein the hypopnea or apnea during sleep is obstructive hypopnea or obstructive apnea, or central nervous system hypopnea or central nervous system apnea.
4. The agent for use as set out at 1 for the prophylaxis or treatment of sleep apnea syndrome.
5. The agent for use as set out at 1 wherein the respiratory disorder during sleep is a respiratory disorder caused by asthma.
6. The agent for use as set out above wherein the intake or dose is 0.06-2500 mg/kg/day based on the free form of glycine.
7. The agent for use as set out above which is a food for the prophylaxis or palliation of snoring or a respiratory disorder during sleep.
8. The agent for use as set out at, wherein the food is in the form of a beverage.
9. The agent for use as set out at 7 or 8, wherein the food is a supplement.
10. The agent for use as set out above which is a diet for the prophylaxis or palliation of snoring or a respiratory disorder during sleep.
11. Use of glycine or a salt thereof in the manufacture of an agent for prophylaxis or treatment of shoring or a respiratory disorder during sleep.

### Brief Description of the Drawings

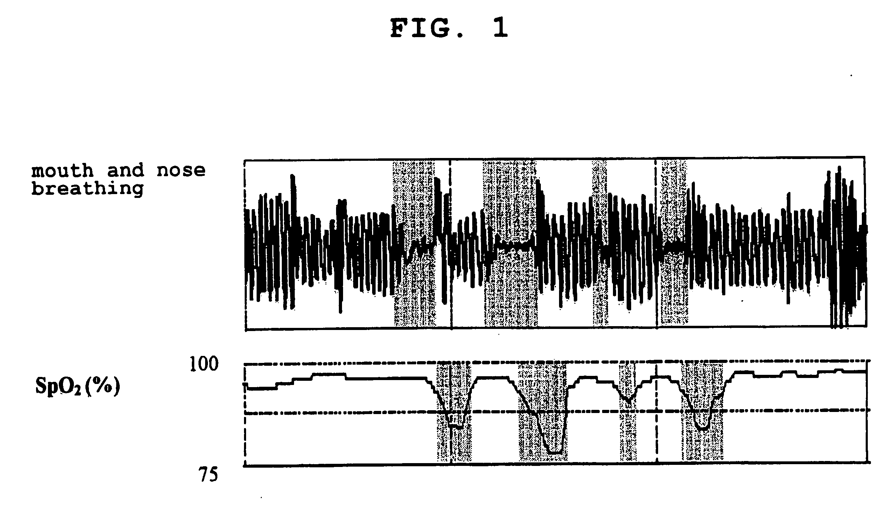

Fig. 1 shows a mouth-nose breathing pattern (upper line) and simultaneously measured profile (lower line) of arterial blood oxygen saturation (SpO₂, % saturation) for 6 min when typical apnea was observed. A temperature increase in a temperature sensor, set on a mouth-nose part, due to expiration and a temperature decrease by inspiration is shown as a mouth-nose breathing pattern. The shadow shows apnea (upper line) and SpO₂ decrease (lower line). An SpO₂ decrease is observed after occurrence of apnea.

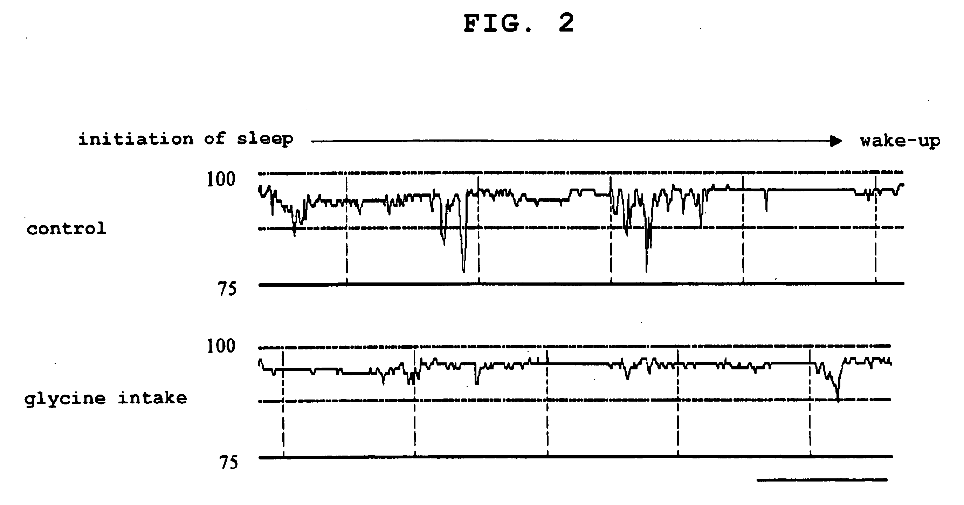

Fig. 2 shows a profile (lower line) of arterial blood oxygen saturation (SpO₂, % saturation) from falling sleep to waking up when a glycine tablet was taken and an SpO₂ profile (upper line) of control free of administration, wherein a bar shows 80 min.

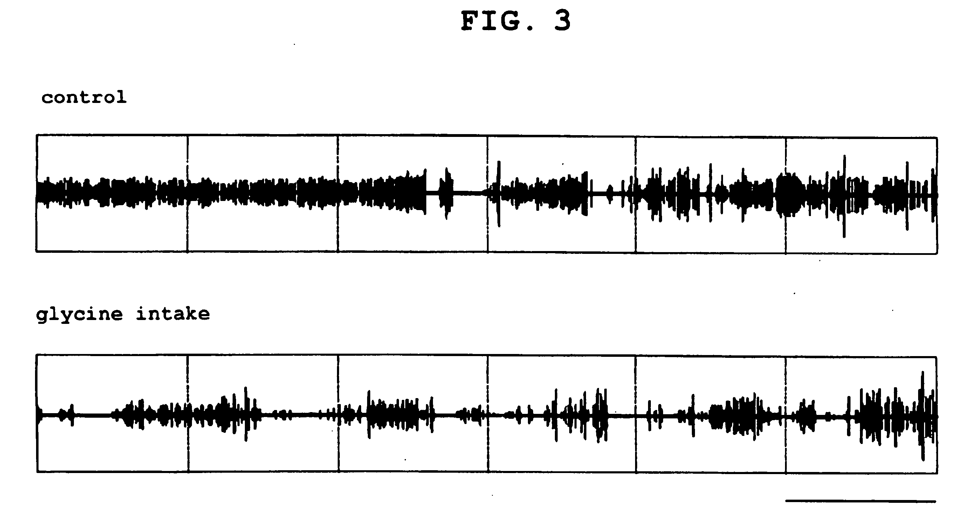

Fig. 3 shows a typical example (lower line) of tracheal sound (snoring noise) when a glycine tablet was taken and a tracheal sound (upper line) of a control free of administration, wherein amplitude shows the level of tracheal sound and a bar shows 5 min.

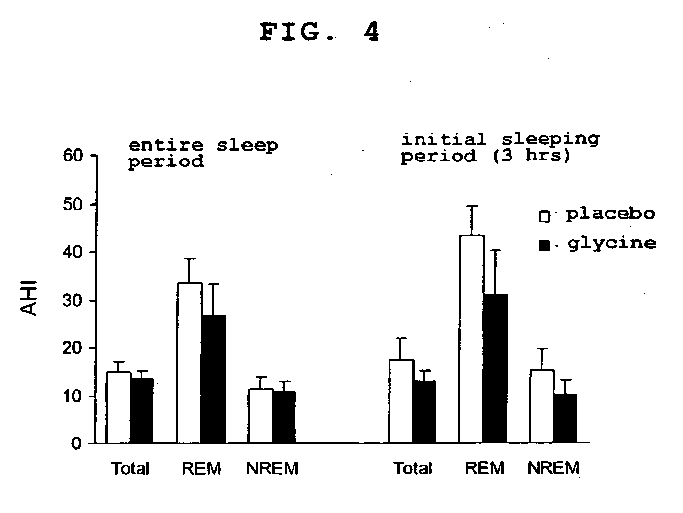

Fig. 4 is a graph showing the results of measurement of AHI during sleep after taking glycine and after taking placebo in Example 2. The left graph of Fig. 4 is an average value of AHI after taking glycine (black column graph) and an average value of AHI after taking placebo (outline column graph) of the entire period (Total) of the rem sleep stage and the nonrem sleep stage, the rem sleep stage (REM) and the nonrem sleep stage (NREM) in the entire sleep stage. The right graph of Fig. 4 shows an average value of AHI after taking glycine (black column graph) and an average value of AHI after taking placebo (outline column graph) of Total, REM and NREM in the early stage of sleep (3 hr).

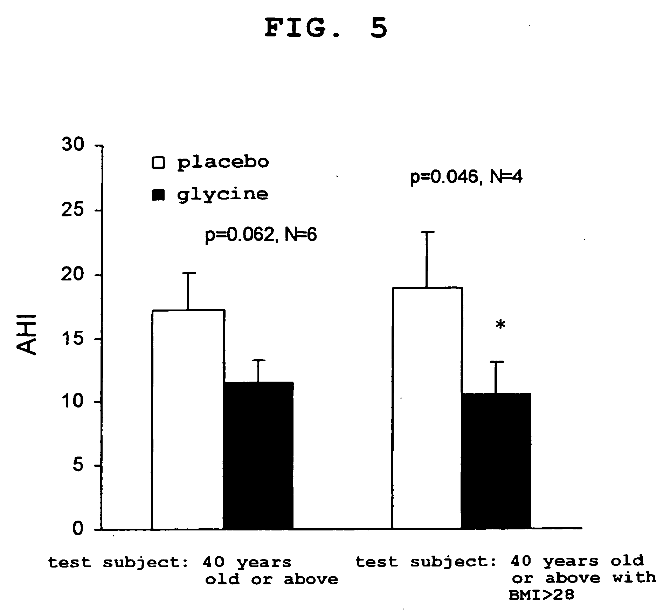

Fig. 5 is a graph showing the results of measurement of AHI during sleep after taking glycine and after taking placebo in Example 2. The left graph of Fig. 5 shows an average value of AHI after taking glycine (black column graph) and an average value of AHI after taking placebo (outline column graph) of test recipients of 40 years old or over and the right graph of Fig. 5 graph shows an average value of AHI after taking glycine (black column graph) and an average value of AHI after taking placebo (outline column graph) of test recipients of 40 years old or over, who shows an obesity index (BMI) exceeding 28, wherein * shows significance.

### Detailed Description of the Invention

The mode of embodiment of the present invention is explained below.

As the snoring and respiratory disorders during sleep that are the application targets of the agent for the prophylaxis or treatment of snoring or a respiratory disorder during sleep of the present invention (hereinafter to be simply referred to as "the agent for the prophylaxis or treatment of the present invention"), snoring, obstructive or central nervous system hypopnea as seen in what is called a sleep apnea syndrome, apnea, as well as hypopnea and apnea caused by other etiology disease such as asthma and the like can be mentioned.

The glycine may be in the form of salts, and the term "glycine", in the present description encompasses salts.

Such salts are not particularly limited as long as they are pharmacologically acceptable and, for example, salts with inorganic acid or organic acid can be mentioned. As the salts with inorganic acid, for example, salts with hydrochloric acid, hydrobromic acid, nitric acid, sulfuric acid, phosphoric acid etc. can be mentioned. As the salts with organic acid, for example, salts with formic acid, acetic acid, trifluoroacetic acid, propionic acid, lactic acid, tartaric acid, oxalic acid, fumaric acid, maleic acid, citric acid, malonic acid, methanesulfonic acid etc. can be mentioned. In addition, salts with base can be also used. As the salts with base, for example, alkali metal salts such as sodium salt, potassium salt etc., alkaline earth metal salts such as calcium salt, magnesium salt etc., and the like can be mentioned.

The agent for the prophylaxis or treatment of the present invention is administered in the form of a known pharmaceutical preparation or a pharmaceutical preparation to be developed in the future, such as the form for oral administration or parenteral administration, and oral administration is convenient and preferable. As the oral agents, solid agents such as powder, granule, tablet, (micro)capsule etc., solutions such as syrup, juice etc., emulsion, suspension and the like can be mentioned. As the parenteral agent, injection, transbowel, transdermal and inhalation preparations can be mentioned. These preparations can be processed into instantaneous or slow acting (sustained-release) preparations.

For production of these preparations, a method known per se or a method to be developed in the future can be appropriately employed. For the production of the preparation, an appropriate pharmaceutically acceptable carrier such as a substance used for general preparations can be used as auxiliary agent according to the dosage form of each preparation. As the substance used for general preparations, for example, excipient, diluent, additive, disintegrant, binder, coating agent, lubricant, gliding agent, glossing agent, flavor, sweetening agent, solubilizer and the like can be mentioned. More concrete examples of the substance used for general preparations include magnesium carbonate, titanium dioxide, lactose, mannitol, dextrin and other sugars, talc, milk protein, gelatin, starch, cellulose (crystalline cellulose) and derivatives thereof, animal and vegetable oils, polyethylene glycol, glyceride, fine silicon dioxide, solvent such as sterilized water, monovalent alcohols (e.g., ethanol etc.) and polyhydric alcohols (e.g., glycerol etc.), vanilla flavor and the like.

The content of the active ingredient (glycine) in the agent for the prophylaxis or treatment of the present invention in the above-mentioned dosage form is not particularly limited and can be appropriately adjusted to contain an amount necessary for providing the efficacy.

The agent for the prophylaxis or treatment of the present invention can be formed as a commercial package containing a written matter describing items relating to the use of the agent for the prophylaxis or treatment.

The recipients of the intake or administration are human and animals (including experimental animals). The intake or dose of glycine is about 0.06-2500 mg/kg/day, more preferably about 0.25-625 mg/kg/day, and still more preferably about 1.25-125 mg/kg/day, based on the free form of glycine.

In the present invention, the daily dose is administered orally or parenterally at once or in 2 to 4 portions at appropriate intervals. For one time administration, it is preferably taken before going to bed.

According to the agent for the prophylaxis or treatment of the present invention, snoring and sleep disorders caused by hypopnea and apnea during sleep can be prevented and the distress due to sleeplessness can be reduced.

According to the agent for the prophylaxis or treatment of the present invention, moreover, snoring or sleep disorders due to hypopnea or apnea during sleep can be prevented, daytime somnolence, scattering of attention, decline of memory, reduced efficiency of work, accident, frustrations, short temper, morning headache, dysthymia, hyposexuality, hallucination, deafness and the like due to sleep disorder, as well as abnormal behavior during night due to sleep disorder can be prevented, and further, social maladjustment due to various symptoms caused by these sleep disorders and anxiety or melancholia can be prevented.

According to the agent for the prophylaxis or treatment of the present invention, moreover, the onset of cerebrovascular disorder, hypertension, polycythemia, pulmonary hypertension, pulmonary heart disease, congestive heart failure, ischemic heart disease, arrhythmia, respiratory failure, sudden death, diabetes, obesity, cognitive impairment, mental disorder and dementia, which are complications of sleep apnea, can be prevented.

The agent for the prophylaxis or treatment of the present invention is effective for improving snoring and respiratory disorders (particularly hypopnea or apnea) in the entire sleep hours from initiation of sleep to wakefulness, and is characterized by the particularly high effectiveness in the early stage of sleep. In addition, the agent for the prophylaxis or treatment of the present invention is effective in remarkably improving snoring or respiratory disorders (particularly hypopnea or apnea) during sleep in individuals of 40 years old or above, particularly individuals of 40 years old or above and with obesity (e.g., obesity of over 28 in BMI).

The "food" of the present invention means food in general (including beverage), general food including what is called health food, food for specified health uses and food with nutrient function claims, as defined in the food with health claims system by the Ministry of Health, Labour and Welfare, and further, supplements.

In the present invention, glycine can be utilized by addition to various processed foods, general foods (e.g., beverage), foods with health claims (e.g., food for specified health uses, food with nutrient function claims) or diets, or as supplements, for the purpose of prophylaxis or palliation of snoring or a respiratory disorder during sleep.

The food in the present invention can be produced by a method known per se using a carrier acceptable as appropriate food, such as various foodstuffs for processing, seasoning, flavor, sweetener and the like as powder, tablet, capsule, drinks and the like. The carrier acceptable as food includes the aforementioned pharmaceutically acceptable carriers.

For production of the food of the present invention, other additives for nutritional support such as vitamins and the like can be used.

The diet in the present invention can be produced by a method known per se using a carrier acceptable as appropriate diet, such as general foodstuffs and the like. The carrier acceptable as diet includes the aforementioned pharmaceutically acceptable carriers. For administration to animals, the aforementioned method for administration to human is employed, or administration upon addition to general diet is employed.

While the amount of glycine to be contained in the food of the present invention is not particularly limited, it is preferably set to such an amount that makes the daily intake fall within the range of the above-mentioned dose of the agent for the prophylaxis or treatment of the present invention.

### Examples

Embodiments of the present invention are explained in detail by referring to Examples.

### Example 1

A test recipient took a glycine tablet and a tablet containing L-cystine as an amino acid other than glycine (hereinafter to be abbreviated as a control tablet) before bedtime and the family member evaluated snoring of the test recipient during sleep. In addition, sleep apnea and arterial blood oxygen saturation (SpO₂) were evaluated using a sleep tester LT-200 manufactured by FUKUDA DENSHI Co., Ltd. (Japan). The test recipient was a 38-year-old healthy male (body weight 82 kg, height 170 cm, body mass index (BMI) 28.3), and frequent occurrence of apnea during sleep was observed by the family member. The test recipient was completely free of regular medication, and had no habit of taking nutritional supplements or nutritional supports. During the test period, no pharmaceutical products, no nutritional supplement and no nutritional support, other than the glycine tablet and the control tablet, were taken. The test recipient took an appropriate number (2-4) of the glycine tablets or the control tablets, and interviewed a family member the next morning as to the presence or absence of snoring and the level thereof, without informing the family member of the kind of the tablets and whether he took the tablets.

### (Preparation of glycine tablet)

Glycine (3,675 g), dextrin (1,725 g) and glyceride (375 g) were mixed, crystalline cellulose (1,500 g) and 70% ethanol at a weight ratio of 22% were added, and the mixture was kneaded and extrusion-granulated. The obtained granulation product was dried to a water content of 2.28% and sized to give 16 mesh PASS particles. Then, fine silicon dioxide (150 g) and vanilla flavor (75 g) were added and mixed. The obtained mixture was tableted under the conditions of 11 mmφ, 500 mg, tableting pressure 2.2 ton, 20 rpm, average hardness 10 kg. By the above operation, 11 mmφ diameter tablets containing 245 mg of glycine per tablet were produced.

### (Preparation of control tablet)

L-cystine (2,625 g), L-teanin (1,050 g), dextrin (1,710 g) and aspartam (15 g) were mixed, crystalline cellulose (1,500 g) and 70% ethanol at a weight ratio of 30% were added, and the mixture was kneaded and extrusion-granulated. The obtained granulation product was dried to a water content of not more than 1.6% and sized to give 16 mesh PASS particles. Then, fine silicon dioxide (37.5 g), glyceride (487.5 g) and vanilla flavor (75 g) were added and mixed. The obtained mixture was tableted under the conditions of 11 mmφ, 500 mg, tableting pressure 2.0 ton, 20 rpm, average hardness 10 kg. By the above operation, 11 mmφ diameter control tablets (cystine teanin tablets) containing 175 mg of L-cystine and 70 mg of L-teanin per tablet were produced.

### (Evaluation of snoring)

The presence or absence of snoring during sleep was evaluated the next morning by a family member into three levels of minus (-: snoring was not heard or like sleep breath), plus (+: medium level snoring), two plus (++: extremely noisy and severe snoring). The presence or absence of snoring when the glycine tablets and control tablets were taken before bedtime, and nothing was taken before bedtime was counted. For comparison between the respective treatments in terms of the results of the snoring evaluation and drinking frequency, χ square test was used and a critical value of p<0.05 was taken as statistical significance.

### (Evaluation of apnea)

The arterial blood oxygen saturation (SpO₂) and air flow in the mouth-nose during sleep were measured using a sleep tester LT-200 manufactured by FUKUDA DENSHI Co., Ltd. The arterial blood oxygen saturation (SpO₂) was measured using a sensor attached to the tip of the second finger of the left hand and when the decrease in the arterial blood oxygen saturation (SpO₂) to not more than 93% lasted for not less than 10 sec was taken as SpO₂ decrease. The number of SpO₂ decrease per one day and per one hour and the minimum SpO₂ of one day were determined. The air flow in the mouth-nose part was measured by a thermocapsule method, and when the decrease in the flow in the mouth-nose part to not more than 50% lasted for not less than 10 sec was defined as hypopnea. The occurrence of SpO₂ decrease along with hypopnea was counted as apnea and the number of apnea per one day and per one hour was determined. Simultaneously, a tracheal sound during sleep was picked up through a mike set near laryngeal prominence.

### (Experiment 1)

For evaluation of whether or not snoring is reduced by taking a glycine tablet, the presence or absence of snoring during sleep was observed for 27 days. After observation period (3 days) free of administration, the presence or absence of snoring was observed after administration of 2 glycine tablets (containing glycine 490 mg) for 9 days, and 4 glycine tablets (containing glycine 980 mg) for 8 days before bedtime and observed for 7 days without administration of glycine tablet (Table 1).

### (Results of Experiment 1)

When the glycine tablet was not administered, extremely annoying snoring (evaluation two plus) was observed for 5 days out of 10 days (frequency 50%) (Table 1). The number of days when evaluation two plus snoring was observed reduced to 2 days out of 9 days (frequency 22%) when glycine tablets (2 tablets) were administered before bedtime, and was not observed when glycine tablets (4 tablets) was administered for 8 days. This decrease was significant as compared to nonadministration of glycine tablet (p<0.05). On the other hand, the number of days when snoring was absent, or of the sleep breath level (evaluation minus) was 3 days out of 10 days (frequency 30%) when the glycine tablet was not administered, but dose-dependently increased to 4 days out of 9 days (frequency 44%) by glycine tablet (2 tablets) administration, and 6 days out of 8 days (frequency 75%) by administration of 4 tablets. Since drinking is known to encourage occurrence of snoring, the number of days of drinking is shown for glycine non-administration, 2 tablet administration and 4 tablet administration (Table 1). Since drinking was highly frequent at the time of glycine 2 tablet and 4 tablet administrations, the reason for suppression of snoring is not reduced drinking but administration of the glycine tablet clearly suppressed the occurrence of snoring.

**[Table 1]**

| glycine (number of tablets taken) | total days of observation | snoring evaluation results | | | number of days of drinking |
|---|---|---|---|---|---|
| | | - | + | ++ | |
| 0 | 10 days | 3 days (30%) | 2 days (20%) | 5 days (50%) | 6 days (60%) |
| 2 | 9 days | 4 days (44%) | 3 days (33%) | 2 days (22%) | 9 days (100% a)) |
| 4 | 8 days | 6 days (75%) | 2 days (25%) | 0 day (0% a)) | 7 days (88%) |

Glycine tablets (0-4 tablets) were administered before bedtime, the presence or absence of snoring was evaluated next morning by a family member. For evaluation results of snoring, the number of days when snoring was not observed (evaluation minus (-)), and the number of days when moderate level of snoring (evaluation plus (+)) and severe level of snoring (evaluation two plus (++)) were observed are shown. In the parentheses, percentages of the number of days when the evaluation was -, +, ++ to the total observation days are shown. Simultaneously, the number of drinking days (percentages to the total number of days of observation in parentheses) is shown. a): Significant (p<0.05) relative to the absence of glycine tablet administration.

### (Experiment 2)

Whether or not the control tablet shows a similar snoring suppressive effect as with the glycine tablet was examined. After an observation period free of administration (5 days) of any tablet, the presence or absence of snoring was observed for 10 days after the control tablets (4 tablets) were administered before bedtime, and for 5 days without administration of the tablet. Thereafter, the presence or absence of snoring was observed for 9 days after the glycine tablets (4 tablets) (containing glycine 980 mg) were administered before bedtime.

### (Results of Experiment 2)

As shown in Table 2, severe snoring (evaluation two plus) was observed for 4 days (frequency 40%) out of 10 days of control tablet administration, and the frequency was not different from the absence of administration of the tablet. In contrast, severe snoring was not observed for 9 days of glycine tablet administration. Conversely, the number of days when the snoring evaluation result was minus was 3 days (frequency 30%) and 2 days (frequency 20%) out of 10 days each of the absence of administration and control tablet administration, respectively. When the glycine tablet was administered, it increased to 6 days (frequency 67%) out of 9 days. From the above results, it is clear that the glycine administration suppressed snoring.

**[Table 2]**

| number of tablets taken | total days of observation | snoring evaluation results | | | number of days of drinking |
|---|---|---|---|---|---|
| | | - | + | ++ | |
| no administration | 10 days | 3 days (30%) | 4 days (40%) | 3 days (30%) | 4 days (40%) |
| glycine tablet | 9 days | 6 days (67% a)) | 3 days (33%) | 0 days (0% a)) | 7 days (78%) |
| control tablet | 10 days | 2 days (20%) | 4 days (40%) | 4 days (40%) | 7 days (70%) |

With regard to each case of glycine tablet administration, control tablet administration and nonadministration before bedtime, the presence or absence of snoring was evaluated the next morning by a family member. For the snoring evaluation results, each number of days when snoring was not observed (evaluation minus (-)), moderate level of snoring (evaluation plus (+)) and severe level of snoring (evaluation two plus (++)) was observed is shown. In the parentheses, percentages of the number of days when the evaluation was -, +, ++ to the total observation days are shown. Simultaneously, the number of drinking days (percentages to the total number of days of observation in parentheses) is shown. a): Significant (p<0.05) relative to the control tablet administration.

When the drinking frequency in Experiment 1 and Experiment 2 was compared between glycine tablet administration and nonadministration, the frequency was higher when associated with administration. Since drinking is generally considered to potentiate snoring, high drinking frequency associated with glycine tablet administration is assumed not to affect the conclusion that "glycine suppresses the occurrence of snoring". To further confirm this, the relationship between the presence or absence of drinking and snoring evaluation results was analyzed (Table 3) with regard to all cases of Experiment 1 and Experiment 2 when glycine tablet was not administered. The number of days when severe snoring (evaluation two plus) was observed was 3 days (frequency 23%) out of 13 days without drinking, but reached 9 days (frequency 53%) out of 17 days of drinking. Conversely, the number of days when snoring was not heard or it was of a sleep breath level (evaluation minus) was 6 days (frequency 46%) out of 13 days without drinking, but significantly decreased to 2 days (frequency 12%) out of 17 days with drinking. From the foregoing, the conventional finding that drinking potentiates snoring was re-confirmed. Therefore, it is clear that the present test results relating to the glycine tablet administration effect were not brought about by the difference in the drinking frequency but because "glycine administration suppressed snoring".

**[Table 3]**

| drinking | total days of observation | snoring evaluation results | | |
|---|---|---|---|---|
| | | - | + | ++ |
| no | 13 days | 6 days (46%) | 4 days (31%) | 3 days (23%) |
| yes | 17 days | 2 days (12% a)) | 6 days (35%) | 9 days (53%) |

The snoring evaluation results of the presence and absence of drinking are shown. For the snoring evaluation results, each number of days when snoring was not observed (evaluation minus (-)), moderate level of snoring (evaluation plus (+)) and severe level of snoring (evaluation two plus (++)) was observed is shown. In the parentheses, percentages of the number of days when the evaluation was -, +, ++ to the total observation days are shown. a): Significant (p<0.05) relative to the absence of drinking.

### (Experiment 3)

A sleep apnea suppressing effect of glycine was evaluated. The air flow in the mouth-nose part during sleep and arterial blood oxygen saturation (SpO₂) were measured for each case of administration of glycine tablet (4 tablets) before bedtime and no administration and the effects of glycine on decreased SpO₂ and frequency of apnea occurrence were evaluated. A tracheal sound during sleep, which is considered to reflect the snoring sound, was simultaneously recorded.

### (Results of Experiment 3)

As shown in Table 4, administration of glycine tablets resulted in the decrease in the number of apnea per night to 40% as compared to nonadministration of glycine. The frequency of apnea per hour also decreased drastically from 4.5 times to 1.9 times by glycine tablet administration. As shown in Fig. 1, a decrease in the arterial blood oxygen saturation (SpO₂) was associated with apnea. Due to the administration of glycine tablets, frequency of SpO₂ decrease was suppressed to about 30% as compared to nonadministration of glycine (Table 4). Fig. 2 shows a profile of arterial blood oxygen saturation during sleep. Due to the administration of glycine tablets, SpO₂ decrease was remarkably suppressed, which in turn increased the lowest value of arterial blood oxygen saturation as shown in Table 4. For comparison of occurrence of snoring between glycine administration and nonadministration, the tracheal sound was simultaneously monitored, and a typical example is shown in Fig. 3. The tracheal sound at the time of glycine tablet administration tended to be suppressed as compared to the nonadministration control group. The palliation of SpO₂ decrease due to glycine tablet administration is considered to be the result of the suppression of sleep apnea by glycine and this effect is considered to be also related to the suppression of occurrence of snoring by glycine.

**[Table 4]**

| glycine administration (4 tablets) | apnea | | SpO₂ decrease | | |
|---|---|---|---|---|---|
| | (times/ day) | (times/ hr) | (times/ day) | (times/ hr) | lowest value |
| - | 27±10 | 4.5±1.3 | 46±3 | 7.8±0.2 | 80±4 % |
| + | 11±4 | 1.9±0.9 | 14±5 a) | 2.5±1.2 a) | 88±1 % |

Using a sleep tester LT-200, mouth-nose breathing and arterial blood oxygen saturation (SpO₂) during sleep were measured, and the number of apnea and SpO₂ decrease per day and per one hour are shown for each case of tablet nonadministration and glycine tablet administration (4 tablets). In addition, the observed lowest value of SpO₂ during sleep is shown for each case of tablet nonadministration and glycine tablet administration. a): significant against no administration of glycine tablet (p<0.05, student's t-test).

Snoring prevents sleep of surrounding people as well as snorer and could be the cause of daytime fatigue, lack of concentration and, sometimes, a serious accident. A decrease in the arterial blood oxygen saturation due to sleep apnea also stimulates wakefulness, which in turn causes a pathologic sleep disorder. Moreover, it is considered that the decreased arterial blood oxygen saturation becomes one cause of circulatory diseases and the like. In the present Examples, it has been clarified that glycine suppresses the occurrence of snoring and alleviates sleep apnea, and further that glycine markedly suppresses decrease in the arterial blood oxygen saturation during sleep. These effects of glycine provide high quality sleep, which is effective for the prevention of daytime fatigue, enhancement of vitality, reduction of the risk of serious accidents caused by lack of concentration or dozing, and further for the prophylaxis of various diseases caused by sleep apnea. In addition, since the action of glycine is considered to be suppression of airway constriction during sleep, glycine can be effectively used for suppressing decrease in the arterial blood oxygen saturation during sleep, which is due to a disease such as asthma and the like.

### Example 2

For confirmation of the effect of glycine on respiratory disorder during sleep, a double blind crossover test was performed with male 10 adults having sleep apnea syndrome as evidenced by more than 5 of the following AHI (apnea hypopnea index per 1 hr). Glycine (3 g) was administered 1 hr before sleep and, as the placebo, maltose adjusted to an indistinguishable taste was used.

### (Evaluation of hypopnea or apnea)

Sleep polygraph data such as electroencephalogram, electromyogram, arterial blood oxygen saturation and the like during sleep were measured using the Alice system manufactured by Respironics Co., Ltd. (USA). The air flow of the mouth-nose part was measured, and hypopnea was defined to mean a state where a decrease to not more than 50% requires not less than 10 sec to recover to not less than 60%. Furthermore, a decrease in the arterial blood oxygen saturation by not less than 3% associated with hypopnea was taken as apnea. The total of the incidents of apnea and hypopnea per 1 hr was taken as an apnea hypopnea index (AHI) and used as a severity index of respiratory disorder during sleep.

The breathing state during sleep after taking glycine or placebo was measured by the above-mentioned sleeping polygraph examination. As a result, an average value of AHI after taking glycine was lower for any of the entire period (Total) of the rem sleep stage and the nonrem sleep stage, the rem sleep stage (REM) and the nonrem sleep stage (NREM) than the average value of AHI after taking placebo. Namely, as shown in Fig. 4, left graph, the average value of AHI after taking glycine vs. average value of AHI after taking placebo was 13.4 vs. 15.0 for the entire period (Total) of the rem sleep stage and the nonrem sleep stage, 26.8 vs. 33.5 for the rem sleep stage (REM), and 10.8 vs. 11.3 for the nonrem sleep stage (NREM). From the foregoing results, it is clear that the glycine administration improved the respiratory disorder during sleep.

As shown in Fig. 4 right graph, moreover, hypopnea or apnea was particularly frequently observed in an early stage of sleep (3 hr) after taking placebo, but the respiratory disorder in the early stage of sleep was improved by glycine administration.

Particularly, in the test recipients of 40 years old or over (6 cases), a remarkable effect of improving respiratory disorders by glycine administration was observed over the entire sleep stage. Namely, as shown in Fig. 5 left graph, an average value of AHI after taking glycine vs. average value of AHI after taking placebo was 11.5 vs. 17.2 (p=0.062). Specifically, in the test recipients of 40 years old or over, who show an obesity index (BMI) exceeding 28 (4 cases), a significant effect of improving respiratory disorders by glycine administration was observed. Namely, as shown in Fig. 5 right graph, an average value of AHI after taking glycine vs. average value of AHI after taking placebo was 10.6 vs. 18.9 (p=0.046). The test was a t-test (paired t-test) and p values of less than 0.05 were taken to be significant.

The foregoing results demonstrate that glycine is effective for improving respiratory disorders over the entire sleep stage, and particularly highly effective in the early stage of sleep. It has been also demonstrated that glycine has an effect of remarkably improving respiratory disorders during sleep in the test recipients of 40 years old or over, particularly the test recipients of 40 years old or over, who show an obesity (BMI>28).

In general, sleep is divided into a nonrem sleep and a rem sleep, and it is said that the occurrence of hypopnea/apnea may vary depending on the state of sleep; in other words, the frequency may change between rem sleep and nonrem sleep. Rem sleep is characterized by rapid eye movements during sleep. Rem sleep and nonrem sleep are each known to play an important role for maintenance of health.

As is clear from the results of Example 2 (Fig. 4), improvements in the respiratory disorders by glycine administration were observed in both periods of rem sleep stage and nonrem sleep stage in both the entire sleep stage and an early stage of sleep (3 hr).

### Industrial Applicability

The agent for the prophylaxis or treatment of the present invention effectively prevents snoring and remarkably suppresses sleep apnea. Therefore, it is effective for the prophylaxis and/or treatment of what is called a sleep apnea syndrome, and moreover, effective for the prophylaxis and/or treatment of respiratory disorders caused by other diseases such as asthma and the like.

As a result of improvement in hypopnea/apnea by the agent for the prophylaxis or treatment of the present invention, high quality sleep can be obtained, which in turn prevents daytime somnolence during sleep, scattering of attention, decline of memory, reduced efficiency of work, accident, frustrations, short temper, morning headache, dysthymia, hyposexuality, hallucination, deafness and the like due to sleep disorder, as well as abnormal behavior during night due to sleep disorder, and further, social maladjustment due to various symptoms caused by these sleep disorders and anxiety or melancholia.

In other words, as a consequence, it prevents daytime fatigue, enhances vitality, reduces risk of serious accidents caused by lack of concentration or by dozing, and further prevents various diseases such as cerebrovascular disorder, hypertension, ischemic heart disease, arrhythmia, sudden death etc. caused by sleep apnea.

Since glycine which is the active ingredient of the agent for the prophylaxis or treatment of the present invention, has established safety, the agent for the prophylaxis or treatment of the present invention is highly safe and can be used not only for pharmaceuticals but also food and diet.

## Claims

1. An agent for use in the prophylaxis or treatment of snoring or a respiratory disorder during sleep, the agent comprising glycine or a salt thereof.

2. The agent for use of claim 1, wherein the respiratory disorder during sleep is hypopnea or apnea.

3. The agent for use of claim 2, wherein the hypopnea or apnea during sleep is obstructive hypopnea or obstructive apnea, or central nervous system hypopnea or central nervous system apnea.

4. The agent for use of claim 1 for the prophylaxis or treatment of sleep apnea syndrome.

5. The agent for use of claim 1 wherein the respiratory disorder during sleep is a respiratory disorder caused by asthma.

6. The agent for use of any one of the preceding claims wherein the intake or dose is 0.06-2500 mg/kg/day based on the free form of glycine

7. The agent for use of any one of the preceding claims which is a food for the prophylaxis or palliation of snoring or a respiratory disorder during sleep.

8. The agent for use of claim 7, wherein the food is in the form of a beverage.

9. The agent for use of claim 7 or claim 8, wherein the food is a supplement.

10. The agent for use of any one of the preceding claims which is a diet for the prophylaxis or palliation of snoring or a respiratory disorder during sleep.

11. Use of glycine or a salt thereof in the manufacture of an agent for the prophylaxis or treatment of shoring or a respiratory disorder during sleep.

## Patentansprüche

1. Mittel zur Verwendung in der Prophylaxe oder Behandlung von Schnarchen oder einer Atmungsstörung während des Schlafens, wobei das Mittel Glycin oder ein Salz davon enthält.

2. Mittel für die Verwendung nach Anspruch 1, wobei die Atmungsstörung während des Schlafens Hypopnoe oder Apnoe ist.

3. Verfahren für die Verwendung nach Anspruch 2, wobei die Hyponoe oder Apnoe während des Schlafens obstruktive Hypopnoe oder obstruktive Apnoe oder eine Zentralnervensystemhypopnoe oder Zentralnervensystemapnoe ist.

4. Verfahren für die Verwendung nach Anspruch 1 für die Prophylaxe oder Behandlung des Schlafapnoesyndroms.

5. Verfahren für die Verwendung nach Anspruch 1, wobei die Atmungsstörung während des Schlafens eine durch Asthma verursachte Atmungsstörung ist.

6. Mittel für die Verwendung nach einem der vorstehenden Ansprüche, wobei die Aufnahme oder Dosis 0,06 bis 2500 mg/kg/Tag, bezogen auf die freie Form von Glycin, ist.

7. Mittel für die Verwendung nach einem der vorstehenden Ansprüche, welches ein Nahrungsmittel für die Prophylaxe oder Linderung des Schnarchens oder einer Atmungsstörung während des Schlafens ist.

8. Mittel für die Verwendung nach Anspruch 7, wobei das Nahrungsmittel in der Form eines Getränks vorliegt.

9. Mittel für die Verwendung nach Anspruch 8 oder 9, wobei das Nahrungsmittel ein Ergänzungsmittel ist.

10. Mittel für die Verwendung nach einem der vorstehenden Ansprüche, welches eine Diät für die Prophylaxe oder Linderung des Schnarchens oder einer Atmungsstörung während des Schlafens ist.

11. Verwendung von Glycin oder eines Salzes davon in der Herstellung eines Mittels für die Prophylaxe oder Behandlung des Schnarchens oder einer Atmungsstörung während des Schlafens.

## Revendications

1. Agent pour utilisation dans la prophylaxie ou le traitement des ronflements ou d'un trouble respiratoire au cours du sommeil, l'agent comprenant de la glycine ou un sel de celle-ci.

2. Agent pour utilisation selon la revendication 1, où le trouble respiratoire au cours du sommeil est l'hypopnée ou l'apnée.

3. Agent pour utilisation selon la revendication 2, où l'hypopnée ou l'apnée au cours du sommeil est une hypopnée obstructive ou une apnée obstructive, ou une hypopnée du système nerveux central ou une apnée du système nerveux central.

4. Agent pour utilisation selon la revendication 1, pour la prophylaxie ou le traitement du syndrome de l'apnée du sommeil.

5. Agent pour utilisation selon la revendication 1, où le trouble respiratoire au cours du sommeil est un trouble respiratoire provoqué par l'asthme.

6. Agent pour utilisation selon l'une quelconque des revendications précédentes, où la prise ou la dose est de 0,06 à 2500 mg/kg/jour en se basant sur la forme libre de la glycine.

7. Agent pour utilisation selon l'une quelconque des revendications précédentes, qui est un aliment pour la prophylaxie ou la palliation des ronflements ou d'un trouble respiratoire au cours du sommeil.

8. Agent pour utilisation selon la revendication 7, où l'aliment est sous la forme d'une boisson.

9. Agent pour utilisation selon la revendication 7 ou la revendication 8, où l'aliment est un complément.

10. Agent pour utilisation selon l'une quelconque des revendications précédentes, qui est un régime alimentaire pour la prophylaxie ou la palliation des ronflements ou d'un trouble respiratoire au cours du sommeil.

11. Utilisation de la glycine ou d'un sel de celle-ci dans la fabrication d'un agent destiné à la prophylaxie ou au traitement des ronflements ou d'un trouble respiratoire au cours du sommeil.
